(19) Europäisches Patentamt
European Patent Office
Office européen des brevets

(11) Publication number: **0 449 546 B1**

(12) **EUROPEAN PATENT SPECIFICATION**

(45) Date of publication of patent specification: **20.09.95**

(51) Int. Cl.[6]: **C07C 213/02**, C07C 215/76

(21) Application number: **91302567.2**

(22) Date of filing: **25.03.91**

(54) **Process for the preparation of m-aminophenols from resorcinol.**

(30) Priority: **26.03.90 US 498980**

(43) Date of publication of application:
**02.10.91 Bulletin 91/40**

(45) Publication of the grant of the patent:
**20.09.95 Bulletin 95/38**

(84) Designated Contracting States:
**BE DE FR GB IT NL**

(56) References cited:

**EP-A- 0 168 976** **EP-A- 0 197 633**
**EP-A- 0 224 625** **EP-A- 0 321 158**
**DE-A- 2 140 786** **DE-A- 2 426 186**
**DE-B- 1 289 530** **FR-A- 850 743**

**PATENT ABSTRACTS OF JAPAN vol. 10, no. 23 (C-325)(2080) 29 January 1986,& JP-A-60 174751 (MITSUI SEKIYU KAGAKU KOGYO K.K.) 09 September 1985,**

**CHEMICAL ABSTRACTS, vol. 106, no. 7, 16 February 1987 Columbus, Ohio, USA &JP-A-60215654 [MITSUI PETROCHEMICAL INDUSTRIES LTD] 29 OCTOBER 1985 page 600; column 2; ref. no. 49767B**

(73) Proprietor: **INDSPEC CHEMICAL CORPORATION**
**Chamber of Commerce Building**
**411 Seventh Avenue**
**Pittsburgh, PA 15219 (US)**

(72) Inventor: **Dressler, Hans**
**1236 Catalina Drive**
**Monroeville,**
**Pennsylvania 15146 (US)**

(74) Representative: **Bull, Michael Alan et al**
**Haseltine Lake & Co.**
**Hazlitt House**
**28 Southampton Buildings**
**Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the preparation of m-aminophenols from resorcinol. More specifically, the present invention relates to the preparation of m-aminophenols by reacting anhydrous amine compounds selected from ammonia, primary amines or secondary amines with resorcinol in an organic solvent in the presence of certain silica alumina catalysts. In particular, the present invention relates to the preparation of the compound m-aminophenol by reacting anhydrous ammonia and resorcinol in an organic solvent in the presence of a natural or synthetic silica alumina catalyst.

The family of m-aminophenol compounds are well known and well reported in the literature. These compounds are useful as intermediates for preparing dyes, pharmaceuticals, resins and agrichemicals. More recently m-aminophenol compounds, particularly m-aminophenol itself, have found application in the preparation of high performance polymers, particularly in the preparation of intermediates for high performance fibres.

The most well known process for preparing aminophenols is by reacting resorcinol with an aminating agent selected from ammonia, primary amines and secondary amines. The compound m-aminophenol is most commonly prepared by reacting resorcinol with ammonia. The amination of resorcinol with aminating agents selected from ammonia, primary amines and secondary amines is well documented and is the subject of many patents and patent applications.

As an example JP 60,215,654 (Chem. Abs. 1987, 106(7), 4 9767B discloses a process in which resorcinol is reacted with a molybdenum containing composite oxide catalyst.

All of the methods comprise reacting resorcinol with the amine, however, there are many different variations of the reaction. For example, it is well known to react resorcinol with the amine in an aqueous medium. This is undesirable because of the necessity of stripping the water with a concomitant loss of product. In addition, the stability of m-aminophenol in aqueous mother liquors for recycle has been erratic. It has also been suggested to run the amination in various solvents with or without the use of various catalysts. Some of these methods have been somewhat successful, however, they suffer from one or more different drawbacks. For example, some catalysts are very expensive. Some catalysts cannot be recycled without being regenerated and some are difficult to separate from the reaction mixture. Some of the other deficiencies associated with the prior art processes include low conversion of resorcinol per pass, poor selectivity in the conversion to aminophenol with too high conversion to m-phenylenediamines, long reaction times and difficulty in isolating and/or purifying the m-aminophenol.

Accordingly, it is an object of this invention to provide an improved method for aminating resorcinol with ammonia, a primary amine or a secondary amine. It is a further object of this invention to provide a process for reacting resorcinol with ammonia, a primary amine or a secondary amine that combines high conversion of resorcinol to aminophenol compound with low amounts of m-phenylene-diamine by-products and which does not suffer from the other drawbacks of the prior art processes for reacting resorcinol with amines.

According to the present invention there is provided a process for preparing m-aminophenols of the formula

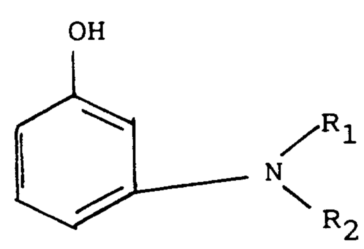

wherein $R_1$ and $R_2$ are independently selected from H and alkyl groups of from 1 to 12 carbon atoms or where $R_1$ and $R_2$ together form a cyclic amine selected from piperidines, pyrrolidines and morpholines, comprising reacting resorcinol with an aminating agent selected from ammonia, primary amines and secondary amines represented by the formula

$HNR_1R_2$

wherein $R_1$ and $R_2$ are as defined above; wherein the improvement comprises running the reaction in an inert organic solvent selected from phenols and alkyl phenols with an anhydrous aminating agent in the

presence of a natural or synthetic silica alumina catalyst.

In accordance with the present invention, it has been found that m-aminophenols can be prepared from resorcinol and amines in high yield, with minimal formation of undesired by-products and with minimization of effluent for disposal. More particularly, the present invention comprises an improved process for reacting resorcinol with an aminating agent selected from ammonia, primary amines and secondary amines by running the reaction under anhydrous conditions in an inert organic solvent and in the presence of an aluminosilicate catalyst. The catalyst is a natural or synthetic zeolite or an activated clay which may be recycled.

Fig. 1 is a flow diagram illustrating schematically a process for preparing m-aminophenol from resorcinol and ammonia in accordance with the preferred embodiment of the present invention.

The present invention is directed to an improved process for preparing m-aminophenol compounds by reacting resorcinol with an aminating agent selected from ammonia, primary amines and secondary amines. The improvement comprises running the reaction with an anhydrous aminating agent in an inert organic solvent and in the presence of an aluminosilicate catalyst.

The aminating agents useful in the present invention are selected from ammonia, primary amines and secondary amines and are represented by the following general formula (a):

(a) $NHR_1R_2$

where $R_1$ and $R_2$ are independently selected from H and alkyl groups of 1 to 12 carbon atoms. The alkyl groups may be primary, secondary, tertiary or cycloalkyl groups. $R_1$ and $R_2$ may together form a cyclic amine selected from piperidine, pyrrolidine, morpholine and alkyl derivatives thereof. The preferred aminating agent is selected from ammonia and primary or secondary amines having alkyl groups of 1 to 12 carbon atoms. The most preferred aminating agent is ammonia.

The aminating agents are reacted with resorcinol to form m-aminophenol or N-alkyl substituted m-aminophenol compounds represented by the following general formula (b):

(b)

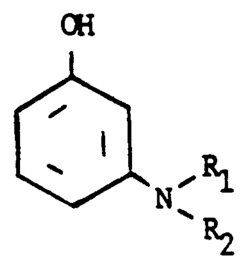

where $R_1$ and $R_2$ are as defined above.

In accordance with the present invention it is important that the aminating agent be anhydrous with the preferred aminating agent being anhydrous ammonia. If the reaction is run using an aqueous medium there is a resulting loss of product and a decrease in the stability of the product. Small amounts of water can be tolerated. Amounts of water exceeding 20 percent by weight based on the weight of resorcinol should be avoided.

In accordance with the present invention the reaction is performed in a suitable inert organic solvent. Examples of suitable solvents are the phenols such as phenol itself, cresols, xylenols and other alkyl phenols. It is important to note that the phenol solvents are not aminated under the conditions of the present process. One of the preferred solvents is phenol and the phenol is not aminated under the conditions of the present process, i.e., no aniline is detectable in the catalyzate.

In accordance with the present invention the reaction must be run in the presence of certain aluminosilicate catalysts. Examples of suitable catalysts are the zeolite (molecular sieve) catalysts which have a high silica/alumina ratio of from 2 to 100, which are preferably in the acid or ammonium-exchanged form such as Union Carbide's LZ M-8 (mordenite structure) and LZ Y-62 (Y-type molecular sieve). Other suitable catalysts include Union Carbide's SK-500 (a rare earth exchanged zeolite), and activated clays (aluminosilicates) such as United Catalyst's montmorillonite K-306 and Filtrol/Harshaw's Filtrol 13 or Filtrol 24 (beidellite activated clay). The amount of catalyst employed will depend on the particular catalyst but generally from 10 to 100 parts by weight of the resorcinol is employed.

The resorcinol and aminating agent are reacted in molar ratios of resorcinol to aminating agent of from 1:1 to 1:5 and preferably from 1:1.5 to 1:3.0. Ratios outside of these ranges may be employed but are not

desirable. For example, ratios of less than 1:1 decrease the yield of desired product without any increase in reactivity or selectivity. In addition, ratios of greater than 1:5 do not increase the yield, reactivity or selectivity. Such excess aminating agent also creates a handling problem.

The resorcinol and aminating agent are reacted at temperatures of between 175°C and 275°C, preferably between 200°C and 240°C. If the temperature is too low, the reaction rate is low and the conversion is low. If the temperature is too high, the formation of undesirable by-products increases, i.e., the selectivity to m-aminophenol decreases. The reaction is run at autogenous pressures.

When the reaction is complete as evidenced by such methods as spectroscopic or chromatographic analysis, the crude m-aminophenol solution is isolated by filtration. The catalyst is then washed, the solvent stripped from the combined filtrate and wash, and the crude m-aminophenol is flash distilled and recrystallized from a suitable medium such as methanol, ethanol (95 or 100%), butanol or solvent mixtures such as toluene/butanol, to give white m-aminophenol of high purity and stability.

In accordance with the present invention it has been found that the catalyst can be recycled many times before it needs regenerating. It has also been found that the unreacted aminating agent and solvent can readily be recycled without any deleterious effect on the product or process. Thus, the overall process is economical and environmental problems are minimized.

In Fig. 1, there is illustrated a flow diagram of a preferred process for making m-aminophenol in accordance with the present invention. Referring now to Fig. 1, resorcinol through conduit 2, phenol via conduit 4 and anhydrous ammonia via conduit 6 are fed to reactor 8 where they are contacted with catalyst (Filtrol-13-LM) from conduit 10 and a recycle stream comprising resorcinol, m-aminophenol and m-phenylenediamine via conduit 13. The reaction is conducted at 220°C ±5°C at a pressure of $1240.2 \times 10^3$ Pa to $1515.8 \times 10^3$ Pa (guage pressure of 180 to 220 lbf/in$^2$) for 5 hours. After reaction is complete, the reaction mixture is cooled to 50-150°C and the residual pressure is released. Excess ammonia can be vented at a higher temperature such as 150°C and recycled via conduit 6. The charge is then passed via conduit 11 to filter 12 and filtered at 50-150°C. The catalyst (wet cake) is washed with molten phenol and recycled via conduit 14 to reactor 8. The combined filtrate and wash are passed via conduit 16 to atmospheric stripping unit 18 where the water formed in the reaction is removed as a water/phenol azeotrope at about 100°C, then the remainder is stripped under vacuum to 150°C (pot) at $26.66 \times 10^2$ Pa (20 torr) to remove the phenol, b.p.-86°C $26.66 \times 10^2$ Pa (20 torr). The phenol is recycled both to filter 12 and reactor 8 via conduit 24. From the phenol stripping unit, the residual crude m-aminophenol is passed via conduit 20 to vacuum distillation unit 22 where a m-aminophenol fraction is recovered at 160-163°C (head) and $13.33 \times 10^2$ Pa (10 torr). The residue is recycled to reactor 8 via conduit 26. The crude reaction product contains resorcinol, m-aminophenol and m-phenylenediamine and is passed from distillation unit 22 via conduit 28 to crystallizer 30 where it is treated with an equal amount of hot n-butanol from conduits 32 and 34. After cooling to 25°C, the slurry is passed via conduit 36 to filter 38 and the resultant white solids passed via conduit 46 to vacuum drier 48 operated at ca. 100°C, $26.66 \times 10^2$ Pa (20 torr) where 97-98% pure m-aminophenol is recovered (m.p. 120-121°C). The combined filtrate and wash from filter 38 are passed via conduit 42 to solvent stripper 44, the recovered n-butanol passed via conduit 34 to crystallizer 30 and also used to wash the filter cake at filter 38, and the residue comprising resorcinol, m-aminophenol and m-phenylenediamine recycled to reactor 8 via conduit 13. The wet crystals from filter 38 are passed via conduit 46 to drier 48 and vacuum dried to produce high purity m-aminophenol and the n-butanol recovered from the drier is recycled via conduit 50 to solvent stripper 44.

The following examples serve to further illustrate the invention and the preferred embodiments thereof. All parts and percentages in the examples and elsewhere in the specification and claims are by weight unless otherwise indicated.

## EXAMPLE 1

A 3.78 L (1-gal.) autoclave was charged with 440.0g (4.0m) resorcinol, 1600 ml. of xylene, 220.0g of Union Carbide catalyst LZM-8 powder (a synthetic zeolite with mordenite structure, ammonium ion exchanged) and 204.0g (12.0m) of anhydrous ammonia. The autoclave was sealed, stirred, heated to 220°C and held at 217-220°C for 5 hrs. The reactor was then cooled to 25°C, vented, discharged, and the product filtered. The catalyst cake was washed with methanol and the combined filtrate and wash was stripped to final conditions of 110°C (pot)/$26.66 \times 10^2$ Pa (20 torr). The dark-coloured residue, by Liquid Chromatographic (LC) analysis, contained 82 wt. % m-aminophenol, 5.7 wt. % resorcinol, and 9.0 wt. % m-phenylenediamine.

EXAMPLES 2-5

Example 1 was repeated in identical manner, except that the used, dried catalyst from the preceding run was used again in the succeeding runs. The average composition of the product over examples 1-5 was 80.6 wt. % m-aminophenol, 9.0 wt. % resorcinol, and 8.4 wt. % m-phenylenediamine. The average recovery of the crude product over five runs was 96.5 wt. %. Resorcinol conversion averaged 91% per pass; the calculated ultimate yield of m-aminophenol was 88.6%.

The crude product was flash-distilled at 13.33 x $10^2$ Pa (10 torr) to give 95.6 wt. % distillate (light yellow colour) and 3.3 wt. % residue. The flash-distilled product was recrystallized from methanol to give a white, crystalline product, estimated to be 99.3% pure by DSC (differential scanning calorimetry) and 98.8% pure m-aminophenol by LC analysis, m.p. 123.0 - 123.5 ° C.

EXAMPLES 6-14

A series of examples were run using the general procedure set forth in Example 1 but with different ratios of ingredients, solvents, catalysts and reaction conditions. The results for this series are set forth in Table 1.

TABLE 1
Amination of Resorcinol with Ammonia

| Ex. No. | m. R./ m. $NH_3$ | Solvent | Catalyst, Wt. %[a] | Temp., °C | Time, hrs. | Product, Wt. %[d] MAP | R | MPDA |
|---|---|---|---|---|---|---|---|---|
| 6 | 1.0/3.0 | Toluene | LZM-8, 100 | 220-234 | 1.5 | 78 | 8.3 | 5.1 |
| 7 | 1.0/2.0 | Toluene | LZM-8, 25 | 236-242 | 5 | 76 | 11.5 | 7.6 |
| 8 | 1.0/3.0 | Xylene | Mont. K-306, 50 | 237-240 | 4 | 75 | 13 | 6.0 |
| 9 | 1.0/3.0 | Xylene | Mont. K-306, (used)[b] | 237-240 | 4 | 73 | 17.5 | 4.9 |
| 10 | 1.0/3.0 | Xylene | Mont. K-306, (used)[c] | 238-241 | 5 | 79 | 16 | 5.5 |
| 11 | 1.0/2.0 | Phenol | Mont. K-306, 50 | 219-231 | 4 | 71 | 18 | 8.0 |
| 12 | 1.0/2.0 | Xylene | SK-500, 50 | 217-240 | 1.0 | 71 | 17 | 4.0 |
| 13 | 1.0/3.0 | Toluene | LZY-62, 50 | 218-223 | 5 | 65.5 | 22 | 6.2 |
| 14 | 1.0/3.0 | 1,2,4-Tri-methyl-benzene | Fil. 13-LM, 50 | 226-233 | 5 | 72.5 | 12 | 4.0 |

a) Catalyst LZM-8 - Union Carbide, synthetic mordenite; Mont. K-306 = United Catalysts, Montmorillonite Clay; SK-500 = Union Carbide, rare earth exchanged synthetic zeolite; LZY-62 = ammonium exchanged Y-type molecular sieve; Fil. 13-LM = Filtrol Corp., acid clay. The wt. % is based on the resorcinol charged.

b) Used catalyst from run 8.

c) Used catalyst from run 9.

d) MAP = m-aminophenol, R = resorcinol, MPDA = m-aminophenylenediamine.

EXAMPLE 15

A 2-litre autoclave was charged with 220.0 g (2.0m.) of resorcinol, 880.0g of phenol (solvent), 292.4g (4.0m.) diethylamine and 88.0g of Filtrol-13LM (catalyst). The autoclave was sealed, stirred and held at 215-220°C for 5 hrs. The charge was then cooled to ca. 60°C. The autoclave was vented and the catalyst was filtered. The catalyst cake was washed with warm (60°C) phenol. The combined filtrate and wash were stripped of unreacted diethylamine; water formed in the reaction and solvent (phenol) were removed by distillation. The residue was flash-distilled at 13.33 x $10^2$ Pa (10 torr) to give a product containing, by LC (liquid chromatographic) analysis, 38.5 wt. % resorcinol and 62.8 wt. % m-diethylaminophenol.

EXAMPLE 16

In accordance with the general procedure of Example 1, m-pyrrolidinylphenol can be prepared by charging a 2-litre autoclave with 220.0g (2.0m) of resorcinol, 880.0g of phenol (solvent), 284.5g (4.0m) of pyrrolidine and 88.0g of Filtrol - 13LM (catalyst). The autoclave will then be sealed, stirred and held at 215-220°C for 4 hrs. The product would then be filtered, the filtrate stripped of low boilers and flash distilled to provide a product containing m-pyrrolidinylphenol which can be identified by Infra-red/Nuclear Magnetic Resonance (IR/NMR) analysis.

EXAMPLE 17

In accordance with the general procedure of Example 1, a 2-litre autoclave was charged with 330.0g (3.0m) resorcinol, 268.5g (3.6m) diethylamine, 330.0g of phenol, and 132.0g of Filtrol - 13LM. The charge was reacted for 5 hrs. at 220°C and then filtered, the filtrate stripped of low boilers and flash distilled to provide a product containing 61 wt. % m-diethylaminophenol and 33 wt. % resorcinol by LC analysis. Based on the resorcinol converted, a 91 wt. % yield of m-diethylaminophenol was obtained.

EXAMPLE 18

In accordance with the general procedure of Example 1, m-propylaminophenol can be prepared by charging a 2-litre autoclave with 220.0 g (2.0 m) of resorcinol, 880.0 g of phenol (solvent), 236.4 g (4.0 m) of propylamine and 110.0 g of Filtrol 13-LM (catalyst). The autoclave will be sealed, stirred and held at 220°C for 5 hours. The charge will be then cooled, vented, discharged and filtered. The filtrate will be stripped of low boilers, including the phenol solvent, and the crude product vacuum-distilled. This distillate will contain a substantial amount of m-propylaminophenol (by IR/NMR analysis).

EXAMPLE 19

In accordance with the general procedure of Example 1, m-cyclohexylaminophenol can be prepared by charging a 2-litre autoclave with 220.0 g (2.0 m) of resorcinol, 880.0 g of phenol (solvent), 297.6 g (3.0 m) of cyclohexylamine, and 88.0 g of Filtrol 13-LM (catalyst). The autoclave will be sealed, stirred and held at 220°C for 5 hours. The reactor will be cooled, vented and discharged. The catalyzate will be filtered and the filtrate stripped of low boilers, including the phenol solvent. The residual, crude product can be analyzed by IR/NMR methods and will be found to contain a substantial amount of m-cyclohexylaminophenol.

EXAMPLE 20

In accordance with the general procedure of Example 1, m-amylaminoresorcinol can be prepared by charging a 2-litre autoclave with 220.0 g (2.0 m) of resorcinol, 880.0 g of phenol (solvent), 348.7 g (4.0 m) of amylamine, and 88.0 g of Filtrol 13-LM (catalyst). The autoclave will be sealed, stirred and held at 220°C for 5 hours. The reactor will then be cooled, vented and discharged. The catalyzate will be filtered and the filtrate stripped of low boilers, including the phenol solvent. The residue will be anaylzed by IR/NMR techniques and found to contain a considerable amount of m-amylaminoresorcinol, with a good conversion of the resorcinol charged.

## Claims

1. An improved process for preparing m-aminophenols of the formula

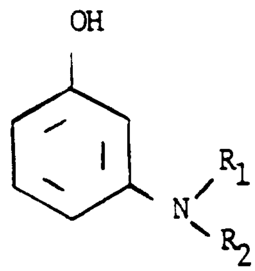

where $R_1$ and $R_2$ are independently selected from H and alkyl groups of from 1 to 12 carbon atoms or where $R_1$ and $R_2$ together form a cyclic amine selected from piperidines, pyrrolidines and morpholines, comprising reacting resorcinol with an aminating agent selected from ammonia, primary amines and secondary amines represented by the formula

$HNR_1R_2$

where $R_1$ and $R_2$ are as defined above; wherein the improvement comprises running the reaction in an inert organic solvent selected from phenols and alkyl phenols with an anhydrous aminating agent in the presence of a natural or synthetic silica alumina catalyst.

2. A process according to claim 1, wherein the catalyst has a silica to alumina ratio of from 2 to 100.

3. A process according to claim 1 or 2, wherein the catalyst is an activated clay.

4. A process according to claim 1 or 2, wherein the catalyst is a natural or synthetic zeolite.

5. A process according to any preceding claim, wherein the inert organic solvent is selected from phenol, cresol or xylenol.

6. A process according to any preceding claim, wherein the ratio of resorcinol to aminating agent is from 1:1 to 1:5.

7. A process according to any preceding claim, wherein the catalyst is employed in an amount of from 10 to 100 parts by weight of resorcinol.

8. A process according to claim 1 for preparing m-aminophenol by reacting resorcinol with ammonia.

9. A process according to claim 8, wherein the catalyst has a silica to alumina ratio of from 2 to 100.

10. A process according to claim 8 or 9, wherein the catalyst is a natural or synthetic zeolite or an activated clay.

11. A process according to any one of claims 8, 9 or 10, wherein the catalyst is employed in an amount of from 10 to 100 parts by weight based on resorcinol.

12. A process according to any one of claims 8 to 11, wherein the ratio of resorcinol to ammonia is from 1:1 to 1:5.

**Patentansprüche**

1. Verbessertes Verfahren zur Herstellung von m-Aminophenol der Formel

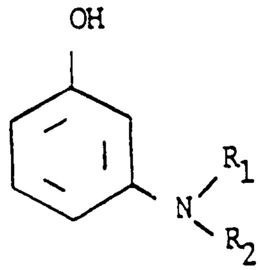

worin $R_1$ und $R_2$ voneinander unabhängig ausgewählt sind aus H und Alkylgruppen mit 1 bis 12 Kohlenstoffatomen, oder
worin $R_1$ und $R_2$ zusammen ein zyklisches Amin bilden, ausgewählt aus Piperidinen, Pyrrolidinen und Morpholinen, wobei das Verfahren umfaßt:
das Umsetzen von Resorcin mit einem amidierenden Mittel, ausgewählt aus Ammoniak, primären und sekundären Aminen, darstellbar durch die Formel

$HNR_1R_2$

worin $R_1$ und $R_2$ wie oben definiert sind; wobei die Verbesserung darin besteht, daß die Umsetzung in einem inerten organischen Lösungsmittel, ausgewählt aus Phenolen und Alkylphenolen, mit einem wasserfreien amidierenden Mittel in Gegenwart eines Katalysators aus natürlichem oder künstlichem Siliciumoxid-Aluminiumoxid erfolgt.

2. Verfahren nach Anspruch 1, wobei das Verhältnis von Siliciumoxid zu Aluminiumoxid im Katalysator 2:100 beträgt.

3. Verfahren nach Anspruch 1 oder 2, wobei der Katalysator ein aktivierter Lehm ist.

4. Verfahren nach Anspruch 1 oder 2, wobei der Katalysator ein natürlicher oder künstlicher Zeolith ist.

5. Verfahren nach irgendeinem vorhergehenden Anspruch, wobei das inerte organische Lösungsmittel ausgewählt ist aus Phenol, Kresol oder Xylol.

6. Verfahren nach irgendeinem vorhergehenden Anspruch, wobei das Verhältnis von Resorcin zu amidierendem Mittel 1:1 bis 1:5 beträgt.

7. Verfahren nach irgendeinem vorhergehenden Anspruch, wobei der Katalysator in einer Menge von 10 bis 100 Gewichtsteilen, bezogen auf Resorcin, eingesetzt wird.

8. Verfahren nach Anspruch 1 zur Herstellung von m-Aminophenol durch Umsetzen von Resorcin mit Ammoniak.

9. Verfahren nach Anspruch 8, wobei das Verhältnis von Siliciumoxid zu Aluminiumoxid im Katalysator 2:100 beträgt.

10. Verfahren nach Anspruch 8 oder 9, wobei der Katalysator ein natürlicher oder künstlicher Zeolith oder ein aktivierter Lehm ist.

11. Verfahren nach irgendeinem der Ansprüche 8, 9 oder 10, wobei der Katalysator in einer Menge von 10 bis 100 Gewichtsteilen, bezogen auf das Resorcin, eingesetzt wird.

12. Verfahren nach irgendeinem der Ansprüche 8 bis 11, wobei das Verhältnis von Resorcin zu Ammoniak 1:1 bis 1:5 beträgt.

## Revendications

**1.** Procédé amélioré de préparation de m-aminophénols de formule :

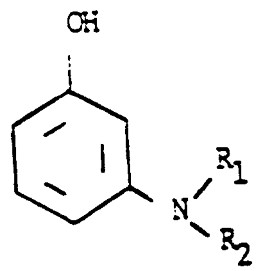

dans laquelle $R_1$ et $R_2$ sont indépendamment choisis parmi les atomes d'hydrogène et les groupes alkyle comportant de 1 à 12 atomes de carbone, ou bien $R_1$ et $R_2$ forment ensemble un résidu d'une amine cyclique choisie parmi les pyrrolidines, les pipéridines et les morpholines,
lequel procédé consiste à faire réagir du résorcinol avec un agent d'amination choisi parmi l'ammoniac, les amines primaires et les amines secondaires et représenté par la formule

$HNR_1R_2$

dans laquelle $R_1$ et $R_2$ sont définis comme ci-dessus,
les améliorations consistant en ce que l'on effectue la réaction dans un solvant organique inerte choisi parmi les phénols et les alkyl-phénols, avec un agent anhydre d'amination, et en présence d'un catalyseur naturel ou synthétique à base de silice et d'alumine.

**2.** Procédé conforme à la revendication 1, dans lequel le rapport de la silice à l'alumine, au sein du catalyseur, vaut de 2 à 100.

**3.** Procédé conforme à la revendication 1 ou 2, dans lequel le catalyseur est une argile activée.

**4.** Procédé conforme à la revendication 1 ou 2, dans lequel le catalyseur est une zéolithe naturelle ou synthétique.

**5.** Procédé conforme à l'une des revendications précédentes, dans lequel le solvant organique inerte est choisi parmi le phénol, les crésols et les xylénols.

**6.** Procédé conforme à l'une des revendications précédentes, dans lequel le rapport du résorcinol à l'agent d'amination vaut de 1/1 à 1/5.

**7.** Procédé conforme à l'une des revendications précédentes, dans lequel on utilise le catalyseur en une quantité de 10 à 100 parties en poids par rapport au résorcinol.

**8.** Procédé de préparation du m-aminophénol, conforme à la revendication 1, dans lequel on fait réagir du résorcinol avec de l'ammoniac.

**9.** Procédé conforme à la revendication 8, dans lequel le rapport de la silice à l'alumine, au sein du catalyseur, vaut de 2 à 100.

**10.** Procédé conforme à la revendication 8 ou 9, dans lequel le catalyseur est une zéolithe naturelle ou synthétique ou une argile activée.

**11.** Procédé conforme à l'une des revendications 8, 9 et 10, dans lequel on utilise le catalyseur en une quantité de 10 à 100 parties en poids par rapport au résorcinol.

**12.** Procédé conforme à l'une des revendications 8 à 11, dans lequel le rapport du résorcinol à l'ammoniac vaut de 1/1 à 1/5.

Fig. I

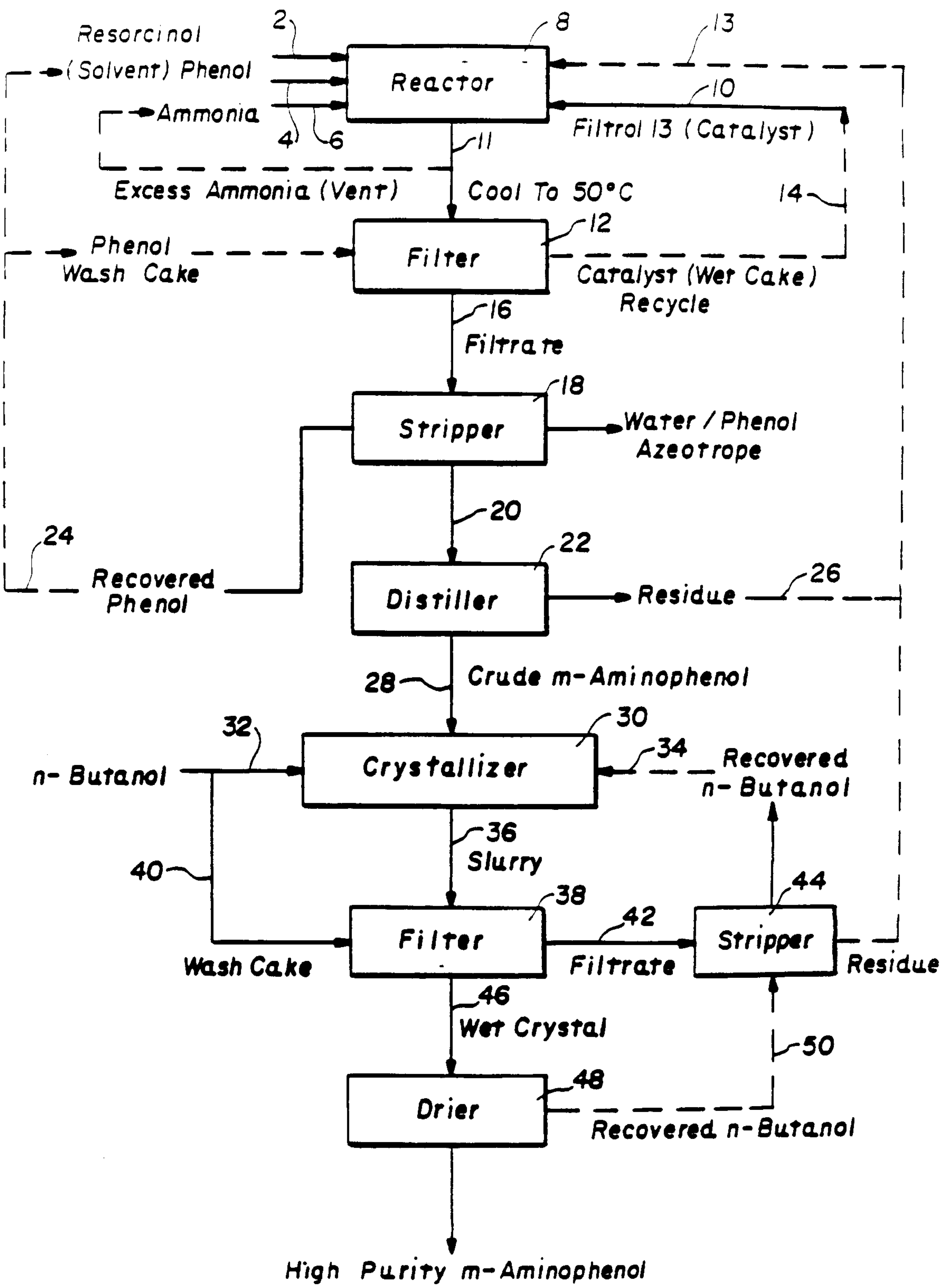
Resorcinol
(Solvent) Phenol
Ammonia
Reactor
Filtrol 13 (Catalyst)
Excess Ammonia (Vent)
Cool To 50°C
Phenol
Wash Cake
Filter
Catalyst (Wet Cake)
Recycle
Filtrate
Stripper
Water / Phenol
Azeotrope
Recovered
Phenol
Distiller
Residue
Crude m-Aminophenol
n- Butanol
Crystallizer
Recovered
n- Butanol
Slurry
Wash Cake
Filter
Filtrate
Stripper
Residue
Wet Crystal
Drier
Recovered n-Butanol
High Purity m-Aminophenol
2
4
6
8
10
11
12
13
14
16
18
20
22
24
26
28
30
32
34
36
38
40
42
44
46
48
50